# EUROPÄISCHE PATENTSCHRIFT

(11) **EP 1 483 017 B1**
(45) Veröffentlichungstag und Bekanntmachung des Hinweises auf die Patenterteilung: **15.10.2008**
(21) Anmeldenummer: 03709630.2
(22) Anmeldetag: 19.02.2003
(51) Int. Cl.: A61N 1/08, A61N 1/05, A61B 5/04

(54) **VORRICHTUNG ZUR LOKALISATION DES ZIELPUNKTES VON ELEKTRODEN ZUR HIRNSTIMULATION, INSBESONDERE ZUR TIEFENHIRNSTIMULATION**
DEVICE FOR LOCATING THE TARGET SPOT OF ELECTRODES USED FOR BRAIN STIMULATION, PARTICULARLY DEEP BRAIN STIMULATION
DISPOSITIF PERMETTANT DE LOCALISER LE POINT CIBLE D'ELECTRODES POUR LA STIMULATION CEREBRALE, EN PARTICULIER POUR LA STIMULATION CEREBRALE PROFONDE

(30) Priorität: 14.03.2002 DE 10211765
(43) Veröffentlichungstag der Anmeldung: 08.12.2004
(73) Patentinhaber: Forschungszentrum Jülich GmbH, 52425 Jülich (DE)
(72) Erfinder: TASS, Peter, 40221 Düsseldorf (DE); SCHIEK, Michael, 52066 Aachen (DE)
(86) Internationale Anmeldenummer: PCT/DE2003/000498
(87) Internationale Veröffentlichungsnummer: WO 2003/077986

(56) Entgegenhaltungen:
- US-A- 6 027 456
- US-A- 6 038 480
- US-A- 6 094 598
- US-A- 6 139 546

## Beschreibung

Die Erfindung betrifft eine Vorrichtung zur Lokalisation des Zielpunktes von Elektroden zur Hirnstimulation, insbesondere zur Tiefenhirnstimulation.

Symptome von neurologischen Erkrankungen, wie z. B. Akinese, Rigor und Tremor werden durch Fehlfunktionen räumlich umschriebener Gebiete des Gehirns hervorgerufen. Diese Symptome können durch Hirnstimulation gelindert oder beseitigt werden, beispielsweise durch Tiefenhirnstimulation. Entscheidend für den Stimulationserfolg ist neben den Stimulationsparametern der Ort der Stimulation.
Die Hirnareale werden nach dem Stand der Technik beispielsweise durch NMR-Aufnahmen oder Computertomographieaufnahmen im groben lokalisiert. Die Genauigkeit dieses Verfahrens ist durch die natürliche Variabilität des anatomischen Aufbaus des Gehirns und durch die begrenzte Auflösung der Verfahren auf Grund der physikalischen Rahmenbedingungen beschränkt. Insbesondere können die für die Fehlfunktion verantwortlichen Neuronenpopulationen klein sein, im Vergleich zu den in den NMR- oder CT-Aufnahmen lokalisierten Regionen. Es ist daher weiterhin notwendig, aufbauend auf den mit diesen Methoden gewonnenen Informationen, eine genauere Bestimmung der Zielpunkte vorzunehmen.

Nach einer bekannten Methode, wie sie beispielsweise in dem Artikel von Benabid A.L., Pollak P., Gervason C., Hoffmann D., Gao D.M., Hommel M., Perret J.E. De Rougemont J. (1991): "Long-term suppression of tremor by chronic stimulation of the ventral intermediate thalamic nucleus" The Lancet 337, 403-406 veröffentlicht ist, wird für jeden Stimmulationsort die Ausprägung der Symptome z. B. die Stärke des Tremors, die Beeinträchtigung der Motorik sowie das Vorhandensein von Rigor und Akinese von einem Neurologen getestet. Hierzu wird in das Gehirn eine Elektrode eingebracht, welche eine fest vorgegebene Zielrichtung besitzt und die in kleinsten Abständen von ca. 1 mm vorwärts und rückwärts bewegt wird. In den jeweiligen Positionen wird mittels der Elektrode eine Stimulation durchgeführt und vom Neurologen die Auswirkung der Stimulation auf die Symptome bei der Reizung an diesem speziellen Punkt neurologisch getestet. Dabei wird der Patient jeweils gebeten, in Abhängigkeit der Stärke der Stimulation zu beschreiben, ob er eine Besserung empfindet. Die durch die Befragung des Patienten erhaltenen Ergebnisse unterliegen jedoch sehr stark subjektiven Einschätzungen und liefern keine objektiven Parameter.

Bei einer anderen Methode, die aus der Veröffentlichung von Levy R., Hutchison W.D., Lozano A.M., Dostrovsky J.O. unter dem Titel "High frequency Synchronization of Neuronal Activity in the Subthalamic Nucleus of Parkinsonian Patients with Limb Tremor" aus dem Journal of Neuroscience 20 (2000) 7766-7775 bekannt ist, werden die für die jeweiligen Kerngebiete des Gehirns charakteristischen Entladungsmuster in der Zielregion, durch einen Elektrophysiologen bzw. einen elekrophysiologisch geschulten Neurologen, mittels Mikroelektrodenableitung detektiert. Hierbei wird nach charakteristischen Frequenzen und/oder Mustern der Entladung gesucht, die mit bestimmten Kerngebieten des Gehirns korrelieren. Mit dieser Methode ist jedoch nur eine grobe Charakterisierung des Befundes möglich, bei der nicht abschließend geklärt werden kann, ob ein Zusammenhang mit der Dysfunktion besteht. Die Methode liefert keine Information zur funktionellen Bedeutung der detektierten Neuronenentladungen. Die Operationen werden um bis zu drei Stunden verlängert.

Die Schrift US 6,027,456 offenbart eine Vorrichtung zur Lokalisation eines Zielpunktes von Elektroden im Rückenmark, mit Hilfe von evozierten Hirnpotentialen.

Die nach dem Stand der Technik bekannten Verfahren sind sehr zeit- und personalintensiv und es fallen keine objektivierbaren Daten an. Es wird lediglich der amplitutenbedingte Stimuluserfolg analysiert. Zusätzlich werden durch das Einbringen der Elektroden kleinste Hirnbereiche verletzt, was dazu führt, dass die Reaktionen auf die Reizungen und die spontane Aktivität in diesen Hirnarealen für einen gewissen Zeitraum geändert werden, bis die entsprechenden Stellen verheilt sind.

Es ist daher die Aufgabe der Erfindung eine Vorrichtung zu schaffen, mit der die optimalen Zielpunkte im Gehirn schnell und besonders objektiv bestimmt werden können. Das Auffinden dieser Zielpunkte soll erleichtert werden.

Ausgehend vom Oberbegriff des Anspruchs 1 wird die Aufgabe erfindungsgemäß gelöst, mit den im kennzeichnenden Teil des Anspruchs 1 angegebenen Merkmalen.

Mit der erfindungsgemäßen Vorrichtung ist es nunmehr möglich, auf schnelle Art und Weise eine objektive Bestimmung des optimalen Zielpunktes im Gehirn vorzunehmen. Die Vorrichtung ermöglicht insbesondere, einen Zusammenhang zwischen dem Zielort im Gehirn und der Dysfunktion herzustellen.

Vorteilhafte Weiterbildungen der Erfindung sind in den Unteransprüchen angegeben.

In der Zeichnung ist ein Schaltschema der erfindungsgemäßen Vorrichtung angegeben.

Es zeigt:

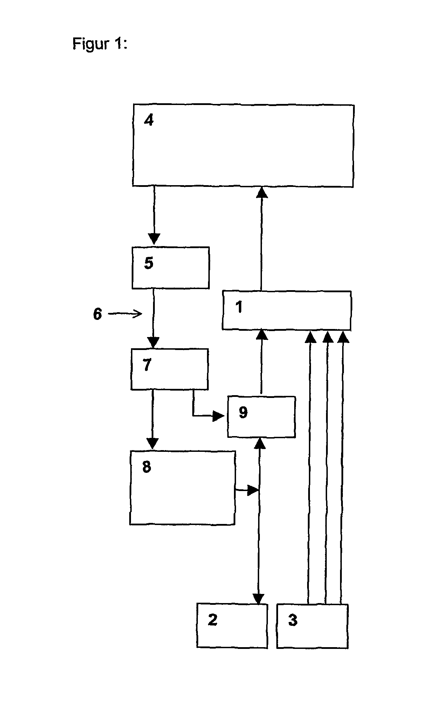

- Figur 1.:: Eine bevorzugte Ausgestaltung der erfindungsgemäßen Vorrichtung als Blockschema.

Die in Figur 1 dargestellte erfindungsgemäße Vorrichtung umfasst einen Trennverstärker (1), an den mindestens eine Elektrode (2) sowie Sensoren (3) zur Erfassung von physiologischen Messsignalen angeschlossen sind. Der Trennverstärker steht weiterhin mit einer Einheit (4) zur Signalverarbeitung und Steuerung in Verbindung, welche an einen optischen Sender für die Stimulation (5) angeschlossen ist. Der optische Sender (5) ist über Lichtwellenleiter (6) mit einem optischen Empfänger (7) verbunden, welcher mit einer Stimulatoreinheit (8) zur Signalerzeugung in Verbindung steht. Die Stimulatoreinheit (8) für die Signalerzeugung steht mit der Elektrode (2) in Verbindung. Am Eingangsbereich der Elektrode (2) in den Trennverstärker (1) befindet sich ein Relais (9) oder ein Transistor.

Die erfindungsgemäße Vorrichtung kann grundsätzlich auf drei verschiedene Arbeitsweisen betrieben werden:
- Die Elektrode (2) misst die neurologische Aktivität, z. B. das lokale Feldpotential (LFP),wobei
   a) ohne oder
   b) mit Stimulation der Zielregion oder
   c) alternierend mit und ohne Stimulation der Zielregion gearbeitet wird.
- Die Sensoren (3) messen ein elektrophysiologisches Merkmal, welches mit der über die Elektrode (2) abgeleiteten neuronalen Aktivität in funktionellem Zusammenhang steht, wie z. B. die neuronale Aktivität einer anderen Hirnregion, den peripheren Tremor oder Herzfrequenz, Atemfrequenz, Durchblutung, Blutdruck, Blutgase.

Im Falle b) erfolgt die Stimulation über die Makro- oder Mikroelektroden (2), welche auch die neuronale Aktivität ableiten.

Erfindungsgemäß wird mit der Vorrichtung sowohl die Entladung in der Zielregion als auch ein physiologisches Merkmal, welches mit der neuronalen Aktivität in der Zielregion in Verbindung steht, gemessen. Hierzu werden die Signale, welche über die Elektrode (2) im Gehirn aufgenommen werden, an den Trennverstärker (1) geleitet und mit den Signalen der Sensoren (3) in Bezug gesetzt.

Als Sensoren (3) können beispielsweise mindestens eine Komponente aus der Gruppe epikkortikale Elektroden, EEG-Skalp-Elektroden, Tiefenelektroden, Hirnelektroden, periphere Elektroden, z. B. zum Messen von Muskelaktivität oder Herzfrequenz, Akzelerometer oder ein Thermistor zur Messung der Atemfrequenz eingesetzt werden. Es können auch zwei oder mehrere gleichartige Elektrodentypen eingesetzt werden.

Erfindungsgemäß ist es hiermit möglich, durch bi- bzw. multivariate Datenanalyse z. B. durch Analyse der Phasensynchronisation oder Kohärenz eine Funktionalität zwischen der neuronalen Aktivität in einem Zielpunkt des Gehirns mit einer Aktivität an einem anderen Körperteil oder an einer anderen Stelle des Gehirns festzustellen. Mit dem Trennverstärker (1) ist es im einfachsten Fall möglich, über wenigstens eine Elektrode (2), an deren Ende eine Potentialdifferenz gemessen werden kann, ein Messsignal aus dem Gehirn zu empfangen und erfindungsgemäß mit mindestens einem weiteren über die Sensoren (3) registrierten Messsignal in Bezug zu setzen. Für diese einfache Ausführungsform gemäß der oben angeführten Fallgestaltung a) wird kein Relais oder kein Transistor (9) benötigt.

Bei der Elektrode (2) handelt es sich um mindestens zwei Drähte, an deren Enden eine Potentialdifferenz gemessen oder angelegt werden kann. In einer weiteren Ausführungsform kann Elektrode 2 auch aus mehr als zwei einzelnen Drähten bestehen, die für die Ermittlung eines Messsignals bzw. die Stimulation im Gehirn herangezogen werden können. Beispielsweise können vier Drähte in einem Leiterkabel untergebracht sein, wobei zwischen verschiedenen Enden eine Potentialdifferenz angelegt oder gemessen werden kann. Hierdurch lässt sich die Größe des untersuchten bzw. stimulierten Zielgebietes variieren. Die Anzahl der Drähte, aus welchen sich die Elektrode zusammensetzt, ist nach oberen Werten hin lediglich durch die damit verbundene Dicke des in das Gehirn einzuführenden Kabels begrenzt, so dass möglichst wenig Hirnmaterial beschädigt werden soll. Handelsübliche Elektroden umfassen vier Drähte, es können jedoch auch fünf, sechs oder mehr Drähte, aber auch nur drei Drähte umfasst sein.

Für den Fall, dass die Elektrode (2) mehr als zwei Drähte umfasst, können mindestens zwei dieser Drähte auch als Sensor (3) fungieren, so dass in diesem Spezialfall eine Ausführungsform vorliegt, bei der die Elektrode (2) und der Sensor (3) in einem einzigen Bauteil vereint sind. Neben diesem Bauteil können zusätzlich nicht mit Elektrode (2) baulich vereinte Sensoren (3) vorhanden sein.

In einer bevorzugten Ausführungsform der Erfindung werden nicht nur lediglich spontane Hirnsignale - also Signale ohne vorhergehende Stimulation gemäß der Fallgestaltung a)- sondern auch Signale verwertet, welche durch eine Stimulation über die Elektroden hervorgerufen worden sind, wie in der Fallgestaltung b). Hierzu werden die vom Trennverstärker (1) aufgenommenen Messsignale der Elektrode (2) sowie der Sensoren (3) an die Einheit (4) zur,Signalverarbeitung und Steuerung weitergegeben.

In einer weiteren bevorzugten Ausführungsform c) wird zwischen den Verfahrensweise a) und b) alterniert. Die für diese Verfahrensweise vorgesehene Vorrichtung umfaßt neben der Elektrode (2), dem Trennverstärker (1), sowie den Sensoren (3) ein Steuerungsgerät (4) zur Signalverarbeitung und Steuerung, welche an den galvanisch entkoppelten Sender (5) mit der Stimulatoreinheit (8) zur Signalerzeugung in Verbindung steht. Bei dieser Ausführungsform ist dem Trennverstärker (1) wiederum ein Relais oder ein Transistor (9) vorgeschaltet. Bei dieser Ausführungsform umfasst die Vorrichtung Mittel zum wahlweisen oder vorprogrammierten Zuschalten der Stimulatoreinheit (8).

Die Einheit zur Signalverarbeitung und Steuerung (4) umfasst Mittel für eine univariate und bivariate Datenverarbeitung zur Kennzeichnung der Frequenzeigenschaften und der Interaktion (Z. B. Kohärenz, Phasensynchronisation und Direktionalität), wie sie beispielsweise in "Detection of n:m Phase Locking from Noisy Data: Application to Magnetoencephalography" von P. Tass, et.al. in Physical Review Letters, 81,3291 (1998) beschrieben ist.

Weiterhin umfasst sie vorzugsweise Mittel zur Visualisierung der Signale und weiterhin vorzugsweise eine Datensicherung. Weiterhin kann die erfindungsgemäße Vorrichtung eine Referenzdatenbank enthalten, welche geeignet ist, Hirnregionen über die registrierte Reizantwort und/oder die spontan registrierten neuronalen Entladungsmuster zu identifizieren. Die Referenzdatenbank kann beispielsweise in die Steuerung (4) integriert sein. Die Signalverarbeitung und Steuerung können auch in verschiedenen Geräten 4, 4a (4a in der Fig. nicht dargestellt) erfolgen. Die verarbeiteten Daten werden an den optischen Sender für die Stimulation (5) weitergegeben, welcher über den Lichtleiter (6) den optischen Empfänger ansteuert. Durch das optische Einkoppeln von Steuersignalen in den optischen Empfänger bei den Verfahrensweise b) und c), wird eine galvanische Entkopplung der Stimulationssteuerung von der Elektrode (2) bewirkt. Dies bedeutet, dass eine Einstreuung von Störsignalen von der Einheit zur Signalverarbeitung und Steuerung (4) in die Elektrode (2) verhindert wird. Weiterhin wird ausgehend vom optischen Sender für die Stimulation (5) über den optischen Empfänger (7) die Relaisschaltung (1) angesteuert, was ebenfalls die Einstreuung von Störsignalen verhindert. Die galvanische Entkopplung muss dabei nicht zwingend durch eine optische Einkopplung der Steuersignale erfolgen, vielmehr können auch andere alternative Steuerungen verwendet werden. Diese können beispielsweise akustische Einkopplungen zum Beispiel im Ultraschallbereich sein, die die Untersuchung nicht stören. Eine störungsfreie Steuerung kann auch beispielsweise unter Zuhilfenahme geeigneter analoger oder digitaler Filter realisiert werden.
Die Relaisschaltung oder der Transistor stellt sicher, dass die neuronale Aktivität unmittelbar nach jedem Stimulus wieder gemessen werden kann ohne dass der Trennverstärker übersteuert. Als optischer Empfänger (7) kommt beispielsweise eine Photozelle in Betracht. Der optische Empfänger gibt die über den optischen Sender für die Stimulation (5) eingegebenen Signale an die Stimulatoreinheit (8) und das Relais (1) weiter. Über die Stimulatoreinheit (8) werden dann gezielte Stimuli über die Elektrode (2) an die potenzielle Zielregion im Gehirn weitergegeben. Die durch die Stimulatoreinheit (8) in die Zielregion gelangten elektrischen Impulse lösen im Gehirn eine Reaktion aus, welche durch die Elektrode (2) wiederum über das Relais an den Trennverstärker (1) weitergegeben werden, der diese Hirnsignale mit physiologischen Merkmalen, welche mit den Sensoren (3) gemessen werden, vergleicht. Somit kann eine direkte Funktionalität zwischen der Relevanz der angepeilten Hirnregion und den damit verbundenen physiologischen Reaktionen hergestellt werden.

Beim Gebrauch der erfindungsgemäßen Vorrichtung werden vorzugsweise folgende Fragestellungen überprüft.
1. Liegt eine pathologische rhythmische Aktivität am Zielort vor? Falls ja, wird die funktionelle Bedeutung dieser rhythmischen Aktivität in folgender Weise untersucht:
2. Ist diese Aktivität phasensynchron oder kohärent mit der Muskelaktivität oder der Aktivität in einem anderen Hirnareal (Messung über eine weitere Tiefenelektrode oder über eine epikortikale Elektrode oder eine EEG-Skalp-Elektrode).
3. Welches ist die Direktionalität der Interaktion zwischen den beiden Messsignalen? Treibt das LFP (lokales Feldpotential) von der Tiefenelektrode die periphere Muskelaktivität oder umgekehrt? Hierdurch wird entschieden, ob es sich beim Tiefenareal um einen Generator des peripheren Tremors handelt, oder ob im Zielareal ein propriozeptives Feedback, das heißt Rückmeldungen vom Funktionszustand z. B. der Muskeln und Sehnen, detektiert wird.
4. Neben der Untersuchung der "spontan", das heißt ohne Stimulation, gewonnenen Signale wird zusätzlich noch die Reaktion der Messsignale (z. B. des Zielareals und der Muskelaktivität) auf standardisierte Testreize objektiv untersucht und klassifiziert. Hierbei wird getestet, wie einerseits die Zielregion und andererseits die Interaktion zwischen Zielregion und einer anderen Hirnregion oder zwischen Zielregion und peripherer Muskelaktivität durch ein standardisiertes Inventar an Stimuli (z. B. kurze Hochfrequenzreize (>100 Hz), periodische Pulse mit einer Pulsfrequenz im Bereich der Tremorfrequenz) moduliert werden. Zur Klassifikation bzw. funktionellen Identifikation des Zielareals werden die Ergebnisse vorzugsweise mit einer Referenz-Datenbank online verglichen.

Die erfindungsgemäße Vorrichtung kann in der Medizin, insbesondere in der Neurologie und Psychiatrie verwendet werden.

## Patentansprüche

1. Vorrichtung zur Lokalisation eines Zielpunktes von Elektroden zur Himstimulation, umfassend mindestens eine Elektrode (2) mit mindestens zwei Drähten, an deren Ende eine einer neuronalen Potentialdifferenz entsprechende Potentialdifferenz gemessen werden kann sowie mindestens einen Sensor (3) zur Erfassung von physiologischen Merkmalen,
wobei sie eine Auswerteeinheit (4) umfasst, die mit einem Trennverstärker (1) in Verbindung steht und die univariate und bivariate Datenverarbeitung zur Kennzeichnung der Frequenzeigenschaften und der Interaktion zwischen Himsignalen und physiologischen Merkmalen, wie Kohärenz und/oder Phasensynchronisation und/oder Direktionalität umfasst.

2. Vorrichtung nach Anspruch 1,
**dadurch gekennzeichnet,**
**dass** der Sensor (3) eine EEG-Skalp-Elektrode, eine epikortikale Elektrode, eine Tiefenelektrode, eine Hirnelektrode, eine periphere Elektrode, eine Muskelelektrode oder eine Kombination von mindestens zwei dieser Sensoren (3) ist.

3. Vorrichtung nach Anspruch 1 oder 2
**dadurch gekennzeichnet,**
**dass** die Auswerteeinheit (4) über Mittel zur Datenvisualisierung verfügt.

4. Vorrichtung nach einem der Ansprüche 1 bis 3,
**dadurch gekennzeichnet,**
**dass** die Auswerteeinheit (4) mit einer Steuereinheit (8) in Verbindung steht, welche die Erzeugung von Stimuli über die Elektroden (2) ermöglicht.

5. Vorrichtung nach einem der Ansprüche 1 bis 4,
**dadurch gekennzeichnet,**
**dass** die Auswerteeinheit (4) mit Mitteln zur galvanisch entkoppelten Einkopplung (5) von Stimuli über die Elektroden (2) in Verbindung steht.

6. Vorrichtung nach Anspruch 5,
**dadurch gekennzeichnet,**
**dass** die Mittel zur galvanischen Einkopplung (5) einen optischen Sender und einen optischen Empfänger umfassen, welche Signale auf die Elektrode (2) übertragen.

7. Vorrichtung nach einem der Ansprüche 1 bis 6,
**dadurch gekennzeichnet,**
**dass** die Auswerteeinheit (4) mit Mitteln in Verbindung steht, welche ein Übersteuern des Trennverstärkers verhindern.

8. Vorrichtung nach Anspruch 7
**dadurch gekennzeichnet,**
**dass** das Mittel zum Verhindern des Übersteuerns des
Trennverstärkers (1) ein Relais oder ein Transistor oder ein elektronischer Filter (9) ist.

9. Vorrichtung nach einem der Ansprüche 1 bis 8,
**dadurch gekennzeichnet,**
**dass** die Elektrode (2) und der Sensor (3) wenigstens teilweise in einem Bauteil umfasst sind.

10. Vorrichtung nach einem der Ansprüche 1 - 9,
**dadurch gekennzeichnet,**
**dass** sie eine Auswerteeinheit (4) mit einer multivariaten Datenverarbeitung umfasst.

## Claims

1. Device for locating a target point of electrodes for brain stimulation, comprising at least one electrode (2) having at least two wires, at the end of which a potential difference corresponding to a neural potential difference can be measured, and at least one sensor (3) for recording physiological features, wherein it comprises an evaluation unit (4) which is connected to an isolation amplifier (1) and which comprises univariate and bivariate data processing to identify the frequency characteristics and the interaction between brain signals and physiological features such as coherence and/or phase synchronisation and/or directionality.

2. Device according to claim 1, **characterised in that** the sensor (3) is an EEG scalp electrode, an epicortical electrode, a depth electrode, a brain electrode, a peripheral electrode, a muscle electrode or a combination of at least two of these sensors (3).

3. Device according to claim 1 or 2, **characterised in that** the evaluation unit (4) has data visualisation means.

4. Device according to one of claims 1 to 3,
**characterised in that** the evaluation unit (4) is connected to a control unit (8) which makes it possible to generate stimuli via the electrodes (2).

5. Device according to one of claims 1 to 4, **characterised in that** the evaluation unit (4) is connected to means for the electrically isolated coupling (5) of stimuli via the electrodes (2).

6. Device according to claim 5, **characterised in that** the means for electrical coupling (5) comprise an optical transmitter and an optical receiver, which transfer signals to the electrode (2).

7. Device according to one of claims 1 to 6, **characterised in that** the evaluation unit (4) is connected to means which prevent an overloading of the isolation amplifier.

8. Device according to claim 7, **characterised in that** the means for preventing the overloading of the isolation amplifier (1) are a relay or a transistor or an electronic filter (9).

9. Device according to one of claims 1 to 8, **characterised in that** the electrode (2) and the sensor (3) are at least partially enclosed in a component.

10. Device according to one of claims 1 to 9, **characterised in that** it comprises an evaluation unit (4) having a multivariate data processing capability.

## Revendications

1. Dispositif de localisation d'un point cible d'électrodes pour la stimulation cérébrale, comprenant au moins une électrode (2) ayant au moins deux fils, auxquelles extrémités on peut mesurer une différence de potentiel correspondant à une différence de potentiel neuronale ainsi qu'au moins un capteur (3) pour enregistrer des caractéristiques physiologiques, celui-ci comprenant une unité d'exploitation (4), qui est connectée à un amplificateur-séparateur (1) et comprend un traitement de données à une variable et à deux variables pour la caractérisation des caractéristiques de fréquence et l'interaction entre les signaux cérébraux et les caractéristiques physiologiques, telles que la cohérence et/ou la synchronisation des phases et/ou la directionnalité.

2. Dispositif selon la revendication 1,
**caractérisé en ce que,**
le capteur (3) est une électrode appliquée au cuir chevelu pour un EEG, une électrode épicorticale, une électrode profonde, une électrode cérébrale, une électrode périphérique, une électrode musculaire ou une combinaison d'au moins deux de ces capteurs (3).

3. Dispositif selon la revendication 1 ou 2,
**caractérisé en ce que,**
l'unité d'exploitation (4) dispose d'un moyen de visualisation de données.

4. Dispositif selon l'une quelconque des revendications 1 à 3,
**caractérisé en ce que,**
l'unité d'exploitation (4) est connectée à une unité de commande (8), laquelle permet l'enregistrement de stimuli via les électrodes (2).

5. Dispositif selon l'une quelconque des revendications 1 à 4,
**caractérisé en ce que,**
l'unité d'exploitation (4) est connectée, grâce à un couplage galvanique (5) aux stimuli via les électrodes (2).

6. Dispositif selon la revendication 5,
**caractérisé en ce que,**
il comprend un émetteur optique et un récepteur optique, lesquels transmettent, grâce à un couplage galvanique (5), des signaux aux électrodes (2).

7. Dispositif selon l'une quelconque des revendications 1 à 6,
**caractérisé en ce que,**
l'unité d'exploitation (4) est connectée à des moyens, lesquels empêchent une saturation de l'amplificateur-séparateur.

8. Dispositif selon la revendication 7,
**caractérisé en ce que,**
le moyen destiné à empêcher la saturation de l'amplificateur-séparateur (1) est un relais ou un transistor ou un filtre électronique (9).

9. Dispositif selon les revendications 1 à 8,
**caractérisé en ce que,**
l'électrode (2) et le capteur (3) sont au moins partiellement inclus dans un composant.

10. Dispositif selon l'une quelconque des revendications 1 à 9,
**caractérisé en ce que,**
l'unité d'exploitation (4) comprend un traitement de données à multiples variables.
